# EUROPEAN PATENT APPLICATION

(11) **EP 1 195 095 A2**
(43) Date of publication of application: **10.04.2002**
(21) Application number: 01307694.8
(22) Date of filing: 11.09.2001
(51) Int. Cl.: A23C 9/123, A23C 9/13, A23C 19/032, A23C 19/05, A23L 3/3463

(54) **Food products with antimicrobial lactic acid bacteria**

(30) Priority: 05.10.2000 GB 0024439
(71) Applicant: St. Ivel Limited, Swindon, Wiltshire SN4 7EF (GB)
(72) Inventor: Griggs, Christopher William, Wootton Bassett, Wiltshire SN4 7EW (GB); Fooks, Laura Janine, Blackwater, Camberley, Surrey GU17 9HD (GB); Gibson, Glenn Robinson, Food Microb. Science Unit, Whiteknights, Reading RG6 6AP (GB)
(74) Representative: Daniels, Jeffrey Nicholas

(57) **Abstract**

A food product containing a combination of (i) a probiotic Lactobacillus plantarum bacterium and (ii) at least one prebiotic oligosaccharide, in an amount so that the combination is capable of inhibiting the growth of food poisoning bacteria.

## Description

The present invention is concerned with a food product for inhibiting food poisoning bacteria and uses thereof.

Probiotics were defined by Fuller (1989) in "Probiotics in man and animals", Journal of Applied Bacteriology 66 365-378 as "live microbial feed supplements which beneficially affect the host animal by improving its intestinal microbial balance". This definition includes some human food supplements in its scope. Probiotic products on the market include powders and tablets for use as dietary supplements, as well as food products containing probiotic bacteria. Cultured dairy products such as yoghurt, fresh cheese, milk drinks, and other fermented milk products are common vehicles for probiotic bacteria. In addition, they are now found also in a variety of other foods.

The most commonly used probiotic bacteria in food products are various strains of species of lactobacilli (e.g. L acidophilus, L casei, L rhamnosus, L reuteri, L johnsonii), enterococci (e.g. E faecium, E faecalis), and bifidobacteria (e.g. B bifidum, B longum, B breve). The beneficial effects of these bacteria are said to include the alleviation of the symptoms of lactose malabsorption (lactose intolerance), the boosting of the body's natural resistance to infectious diseases of the intestinal tract, the lowering of serum cholesterol levels and therefore of coronary heart disease, the suppresion of cancer, the stimulation of the immune system, and the production of vitamins (particularly B vitamins) in the gut. Not all of these effects have been scientifically demonstrated, and in many cases the mechanisms of action are not clear. However, possible mechanisms are thought to include biochemical inhibition or stimulation, competition for nutrients, immune clearance, and competition for adhesion receptors. Probiotic bacteria exert their beneficial effects by surviving the passage through the stomach and small intestine, and establishing themselves and growing in the large intestine.

Substances which stimulate probiotic bacteria have been termed prebiotics. These were defined by Gibson and Roberfroid (1995) in "Dietary modulation of the human colonic microbiota: introducing the concept of prebiotics" Journal of Nutrition 125 1401-1412 as "non-digestible food ingredients that beneficially affect the host by selectively stimulating the growth and/or activity of one or a limited number of bacteria in the large intestine, that can improve the host health". Most are non-digestible carbohydrates such as oligosaccharides. Oligosaccharides that have a prebiotic effect include fructo-oligosaccharides such as inulin and oligofructose, gluco-oligosaccharides, galacto-oligosaccharides, isomalto-oligosaccharides, xylo-oligosaccharides, and soyabean-oligosaccharides.

Various uses of Lactobacillus strains are known.

WO-A-9009398 (Bioinvent International AB) discloses the use of high molecular weight (>8000 Daltons) proteinaceous metabolites produced by Lactobacillus strains, preferably Lactobacillus crispatus (fermentum), that bind to enteropathogenic Escherichia coli. It is suggested that these metabolites could be used to prevent the adhesion, growth and/or survival of pathogens such as E coli, Clostridium, Salmonella, Campylobacter and Streptococcus in humans and livestock.

WO-A-8905849 (Chr Hansens Lab A/S) discloses probiotic lactic acid bacteria, including various strains of Lactobacilli, isolated from the gastro-intestinal tract of pigs. The strains show adherence to pig gastro-intestinal epithelial cells and can be used for treating gastro-intestinal tract infections in pigs or for making fermented milk products for human use.

JP-A-11225749 (Ohayo Nyugyo) discloses a Lactobacillus strain that has antimicrobial activity against food poisoning bacteria such as Salmonella, Shigella, Escherichia, Vibrio and Yersinia, and its use in cultured milk products.

In particular, various uses of Lactobacillus plantarum strains are known.

WO-A-9934679 (Atria OY et al) discloses the use of Pseudomonas bacterial preparations that inhibit salmonellae in food industry premises and processes. Separately, this document discloses the use of lactic acid bacteria preparations containing low molecular weight compounds (<1000 Daltons) for the same purpose. Lactobacillus plantarum and Pediococcus parvulus are the lactic acid bacteria that are mentioned.

WO-A-9629083 (Probi AB) discloses the use of Lactobacillus plantarum having a mannose-specific adhesin in pharmaceutical compositions for inhibiting the adherence of pathogenic bacteria expressing mannose-specific adhesins to the epithelial cell surface. Lactobacillus plantarum 299v and strains that are genotypically very similar are mentioned.

WO-A-9105850 (Tartu University et al) discloses the use of Lactobacillus plantarum 38 for production of preparations for correcting and stabilizing the gastro-intestinal microflora. It is claimed to lower levels of Proteus and Clostridium and to boost levels of Bifidobacterium and Lactobacillus.

EP-B-0554418 (Probi AB) discloses a Lactobacillus strain having the ability to colonize the human intestinal mucosa, the strain being either Lactobacillus plantarum 299 or Lactobacillus casei ssp rhamnosus 271 or a variant of either having an essentially similar restriction endonuclease analysis pattern.

US-A-5895758 (Bio-Energy Systems) discloses a pure culture of Lactobacillus plantarum OM that has proteolytic activity and shows anti-tumour and anti-viral properties.

FR-A-2330334 (Bodielait) discloses the preparation of a food product containing at least one strain of Lactobacillus found in the human gastro-intestinal tract, the lactobacilli being preferably L casei, L acidophilus, L plantarum or L fermenti.

In spite of the prior art, there remains a need to provide a means for effectively inhibiting the growth of food poisoning bacteria in the human or animal body, particularly in the human large intestine.

The present invention aims to address this need and to provide such a means.

Accordingly, the present invention provides a food product containing a combination of (i) a probiotic Lactobacillus plantarum bacterium and (ii) at least one prebiotic oligosaccharide, in an amount so that the combination is capable of inhibiting the growth of food poisoning bacteria.

For the purposes of the present invention, one definition of the phrase "inhibiting the growth of food poisoning bacteria" can be taken to be that at least a six log reduction in counts of food poisoning bacteria is observed as compared with a control (where starch is used in place of the at least one prebiotic oligosaccharide) after:
(a) 1ml of the probiotic Lactobacillus plantarum bacterium is inoculated into a base medium containing 1% of the at least 1 prebiotic oligosaccharide (or starch control) and incubated anaerobically for a period of 24 hours; and
(b) after the 24 hour incubation period, a sample is removed and a dilution series prepared and plated out on an appropriate agar and the plate is incubated for 48 hours after which time colonies are re-numerated.

Generally, a 6 log reduction in counts of food poisoning bacteria would result in a significant enhancement of food safety, when used in conjunction with normal hygiene practices and treatments in food processing aimed at producing foods with low numbers of food poisoning bacteria. As a comparison, in recent guidelines for the processing of chilled foods, a pasteurization-type heat treatment is said to reduce all vegetative pathogens "to an acceptable level (6 log reduction)" (Guidelines for Good Hygienic Practice in the Manufacture of Chilled Foods, 3rd edition; Chilled Food Association; London : CFA : 1997).

The combination of (i) a probiotic Lactobacillus plantarum bacterium and (ii) at least one prebiotic oligosaccharide in a food product for the purpose of inhibiting the growth of, or in other words having an antimicrobial effect against, food poisoning bacteria has been hitherto unknown. Advantageously, a food product according to the present invention will help to improve the health of the consumer of the food by inhibiting the growth of food poisoning bacteria in the consumer. Also the food product will be safe to the consumer insofar as the growth of food poisoning bacteria in the food product itself will be inhibited.

The probiotic Lactobacillus plantarum bacterium used in the present invention may be any Lactobacillus plantarum strain that shows probiotic properties and, in particular, an inhibitory effect on food poisoning bacteria when grown in the presence of at least one prebiotic oligosaccharide. Other characteristics which make a Lactobacillus plantarum bacterium suitable for use in the present invention as a probiotic include acid tolerance which enables the bacterium to survive the acidic conditions in the stomach and bile tolerance which enables the bacterium to survive the conditions in the small intestine and to reach the large intestine where it can become established.

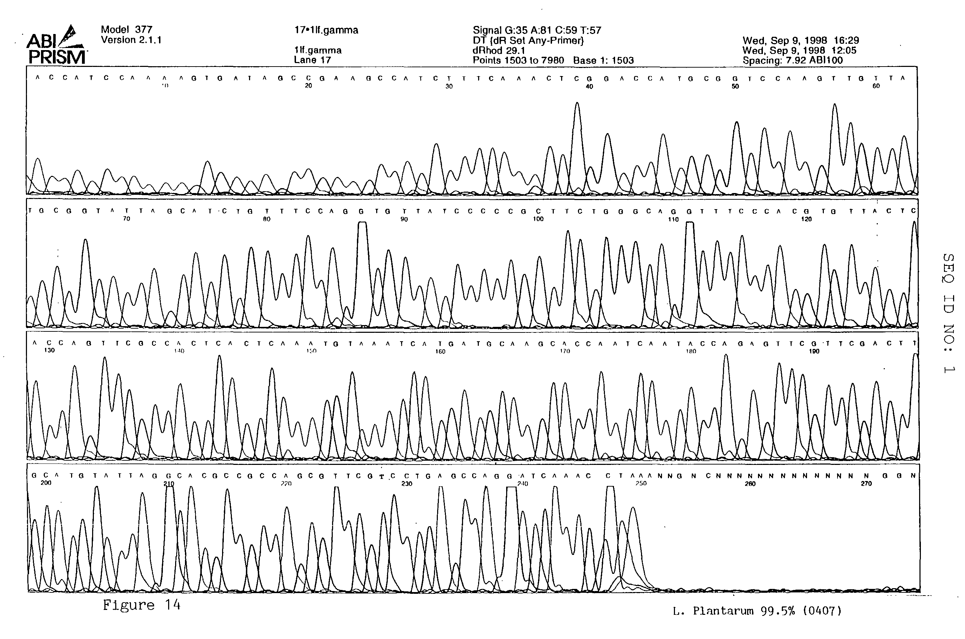

A preferred Lactobacillus plantarum bacterium in accordance with the present invention has a 16S rDNA sequence comprising (i) a sequence as shown in SEQ ID NO: 1 in Figure 14 or (ii) a sequence that is a mutation or variant thereof and which does not substantially reduce the ability of the bacterium to inhibit the growth of food poisoning bacteria in the presence of at least one prebiotic oligosaccharide. A particularly preferred Lactobacillus plantarum bacterium is Lactobacillus plantarum strain LP0407 (NCIMB) No. 41034. This strain was deposited at the National Collection of Industrial and Marine Bacteria (NCIMB Ltd, Aberdeen) on 13 December 1999, under the reference number NCIMB No.41034. This strain possesses all of the advantageous characteristics of a probiotic lactic acid bacterial strain mentioned above.

Preferably, the food product contains at least 10¹² or 10¹³ cells of the probiotic Lactobacillus plantarum bacterium per 1000 litres or 1000 kilograms of the food product. This is to ensure that the minimum required level of 10⁶ viable cells per gram is found in the food product at the end of its normal shelf life. Preferably the Lactobacillus plantarum bacterium is added to the food product at a rate of 10¹³ cells per 1000 litres or 1000 kilograms.

Any oligosaccharide can be used in the food product according to the present invention so long as the combination of the probiotic Lactobacillus plantarum bacterium and the at least one prebiotic oligosaccharide is capable of inhibiting the growth of food poisoning bacteria. Preferred oligosaccharides for this purpose are fructo-oligosaccharides such as oligofructose and inulin, xylo-oligosaccharides and mixtures thereof. In one aspect of the present invention, the at least one prebiotic oligosaccharide comprises a mixture of prebiotic oligosaccharides.

Interestingly, in the absence of at least one prebiotic oligosaccharide and in the presence of starch, a probiotic Lactobacillus plantarum bacterium for use in the present invention, particularly Lactobacillus plantarum LP0407, shows little or no inhibition on the growth of food poisoning bacteria.

Several food poisoning bacteria have been considered in detail for the purposes of the present invention. These food poisoning bacteria are Escherichia coli; Campylobacter jejuni; and Salmonella enteritidis. Some correlation has been observed between the effectiveness of the present food product to inhibit the growth of a particular food poisoning bacterium and the specific at least one oligosaccharide used in the food product. For this reason, it is preferred that the at least one prebiotic oligosaccharide comprises 50% by weight or more, more preferably at least 80%, of oligofructose. This is particularly because it has been found that E coli is inhibited very effectively by a probiotic Lactobacillus plantarum bacterium when in the presence of oligofructose. Similarly, inhibition of the growth of S enteritidis by a probiotic Lactobacillus plantarum bacterium is very effective where the at least one prebiotic oligosaccharide comprises oligofructose, and particularly, where the at least one prebiotic oligosaccharide comprises 80% by weight or more oligofructose.

In the food product, it is preferred that the at least one prebiotic oligosaccharide is present at an amount of up to 10% by weight based on the total weight of the food product. More preferably, the at least one prebiotic oligosaccharide is present in amount in the range 2% to 7%, even more preferably in the range 3% to 6% by weight based on the total weight of the food product. In this regard, where the food product is made first by making a base mixture of which the combination of the probiotic Lactobacillus plantarum bacterium and the at least one prebiotic oligosaccharides form a part, it is the total weight of the base mixture upon which the % by weight of the at least one prebiotic oligosaccharide will be based.

In the present food product, it is usual for the combination of the probiotic Lactobacillus plantarum bacterium and the at least one prebiotic oligosaccharide to be homogeneously dispersed within the food product.

Food products according to the present invention include cultured or non-cultured dairy products. In particular, the present inventors envisage that preferred food products will be yoghurts and cheese products, including fresh cheese products such as fromage frais, cottage cheese and soft cheese; and hard cheese. Non-cultured dairy products and other food products may include milk drinks, desserts, dessert toppings, dips, spreads, and dressings and sauces for use in chilled foods. The Lactobacillus plantarum bacterium will not grow in these food products at the chilled temperatures used for storage. However, the bacterium will survive these temperatures.

The present invention further provides a combined preparation for inhibiting the growth of food poisoning bacteria comprising:
(i) a probiotic Lactobacillus plantarum bacterium; and
(ii) at least one prebiotic oligosaccharide.

The combined preparation may be suitable for use in a dietary supplement in the form of a tablet, capsule or liquid preparation. It is a possibility that the combined preparation may be used in a food product as described above where the probiotic Lactobacillus plantarum bacterium and the at least one prebiotic oligosaccharide in the food product are sourced from the combined preparation.

The present invention further provides a method for making a combined preparation according to the present invention or for making a food product according to the present invention, either method comprising a step of contacting the probiotic Lactobacillus plantarum bacterium with a mixture containing the at least one prebiotic oligosaccharide.

To this end, the present invention still further provides an isolated Lactobacillus plantarum bacterium for use in the preparation of a food product according to the present invention or a combined preparation according to the present invention. Preferably, the isolated Lactobacillus plantarum bacterium has a 16S rDNA sequence comprising (i) a sequence as shown in SEQ ID NO: 1 or (ii) a sequence that is a mutation or variant thereof and which does not substantially reduce the ability of the bacterium to inhibit the growth of food poisoning bacteria. In particular, the present invention provides an isolated Lactobacillus plantarum bacterium which is Lactobacillus plantarum strain LP0407 (NCIMB No. 41034). Mutations or variants of the above sequences may be prepared by any conventional method of mutagenesis of L. plantarum LP0407, including both random and site-directed mutagenesis. Alternatively, the sequences or sub-sequences thereof can be used as hybridization probes to identify candidate strains of L. plantarum which can be further tested to assess whether they are probiotic.

Use of an isolated Lactobacillus plantarum bacterium according to this invention in a food product according to this invention or a combined preparation according to this invention is provided. Further, use of an oligosaccharide for the preparation of a food product according to this invention or a combined preparation according to this invention is provided.

The combined preparation according to the present invention may be used for the manufacture of a composition for inhibiting the growth of food poisoning bacteria. The components of the combined preparation may be incorporated in the composition simultaneously or sequentially, optionally with one or more further additives depending on the exact nature of the composition. In particular, the use of a combined preparation according to the present invention for the preparation of a food product for inhibiting the growth of food poisoning bacteria is envisaged. The food poisoning bacteria include those that cause food poisoning by growing in the large intestine, such as E. coli and C. jejuni. Suitably the inhibition of the growth of food poisoning bacteria occurs in the human or animal body, particularly the human body, even more particularly the human large intestine. As discussed in further detail below, in this embodiment of the invention it is thought that growth of the probiotic Lactobacillus plantarum in the presence of the prebiotic oligosaccharide may occur in the large intestine to the detriment of any food poisoning bacteria there.

The present invention now will be described in more detail, by way of example only, with reference to the attached Figures in which:
Figure 1 shows the change in counts of E. coli 9517 after 24 hours inoculation with L. plantarum 0407 in accordance with Experiment 2(i);
Figure 2 shows the change in counts of C.jejuni 11351 after 24 hours inoculation with L. plantarum 0407 in accordance with Experiment 2(i);
Figure 3 shows the change in counts of S. enteriditis 4444 after 24 hours inoculation with L. plantarum 0407 in accordance with Experiment 2(i);
Figure 4 shows the Lactobacilli counts for E. coli without pH control in accordance with Experiment 2(ii);
Figure 5 shows the coliform counts for E. coli without pH control in accordance with Experiment 2(ii);
Figure 6 shows the Lactobacilli counts for E. coli with pH control in accordance with Experiment 2(ii);
Figure 7 shows the coliform counts for E. coli with pH control in accordance with Experiment 2(ii);
Figure 8 shows the Lactobacilli counts for C. jejuni without pH control in accordance with Experiment 2(ii);
Figure 9 shows the C. jejuni counts for C. jejuni without pH control in accordance with Experiment 2(ii);
Figure 10 shows the Lactobacilli counts for C. jejuni with pH control in accordance with Experiment 2(ii);
Figure 11 shows the C. jejuni counts for C. jejuni with pH control in accordance with Experiment 2(ii);
Figure 12 shows the change in pH for E. coli without pH control in accordance with Experiment 2(ii);
Figure 13 shows the change in pH for C. jejuni without pH control in accordance with Experiment 2(ii); and
Figure 14 shows the 16S rDNA sequence for Lactobacillus plantarum strain LP0407. This sequence is SEQ ID NO: 1.

Without wishing to be bound by theory, there are several proposed mechanisms for the inhibition of the growth of food poisoning bacteria by the combination of the probiotic Lactobacillus plantarum bacterium and the at least one prebiotic oligosaccharide in the present food product.

The mechanism of the inhibition of the food poisoning bacteria has been investigated by a well diffusion assay. The surfaces of TSA plates were inoculated with S enteritidis 4444 to form a lawn of growth and four 5mm diameter wells were cut from each plate. Two wells on each plate were filled with basal medium to act as control and the other two were filled with one of the three samples for each test culture, as follows:-
1. washed centrifuged cells of L plantarum LP0407;
2. cell-free supernatant from L plantarum LP0407 culture; and
3. neutralized cell-free supernatant from L plantarum LP0407 culture.

The test cultures were of L plantarum LP0407 grown in MRS broth or in basal medium with 1% of oligofructose (FOS), xylooligosaccharides (XOS), or inulin (IN). The results were expressed as the diameter in mm of the zone of inhibition of the growth of S enteritidis.

| | Cells | Supernatant | Neutralized Supernatant |
|---|---|---|---|
| MRS broth | 0 | 12.25 | 0 |
| Basal medium + FOS | 0 | 10.25 | 8.5 |
| Basal medium + XOS | 5.75 | 12.75 | 5.75 |
| Basal medium + IN | 0 | 10.75 | 0 |

As would be expected from the known capacity of lactic acid bacteria to produce short chain fatty acids, the greatest inhibition is shown by the supernatant in each case, as this is where these compounds would accumulate. Most lactobacilli produce lactic acid as the end-product of the metabolism of sugars. Since the neutralized supernatant also caused some inhibition, the effect cannot be solely due to the change in pH caused by the production of short chain fatty acids. L plantarum has been reported to produce the bacteriocin lactolin, which might be expected to be associated with the cells, but in only one case did the cell fraction cause inhibition. However the production of a bacteriocin cannot ruled out, as the results of the batch fermentation experiments also confirmed that the inhibitory effects were not due to the change in pH alone.

### EXPERIMENT 1: CHARACTERISATION OF LACTOBACILLUS PLANTARUM LP0407

### (i) Survival under acidic conditions

This was demonstrated by counting the bacteria after 3 hours in hydrochloric acid solutions of various pH values. The results were expressed as the ratio of the natural logarithms of the number of colony forming units per mL after 3 hours and the number of colony forming units per mL at 0 hours.

| | | | | |
|---|---|---|---|---|
| (ln cfu/mL @ 3 hrs)/ | pH 5 | pH 4 | pH3 | pH 2 |
| (ln cfu/mL @ 0 hrs) | 1.053 | 1.061 | 1.000 | 0.475 |

The results show that L plantarum LP0407 grew at pH 4 or pH 5 and survived without growth at pH 3, but that at pH 2 there was a decrease in viable cells, with only a proportion of cells surviving.

### (ii) Bile tolerance

This was demonstrated by growing the bacteria in MRS agar or in milk in the presence of various concentrations of ox gall. The results were expressed as growth rates (optical density units/hr).

| | 0.0% ox gall | 0.3% ox gall | 0.5% ox gall |
|---|---|---|---|
| MRS agar (OD units/hr) | 0.94 | 0.63 | 0.66 |
| Milk (OD units/hr) | 0.65 | 0.80 | 0.55 |

The results show that the growth rate of L plantarum LP0407 was reduced by 30-33% in the presence of ox gall when grown on MRS agar, and by a maximum of 15% when grown in milk.

### (iii) Sequencing

The identity of L plantarum LP0407 was established by biochemical testing using an API 50 CHL kit (TM - Biomérieux) specifically designed for the identification of lactic acid bacteria. The biochemical profile of strain LP0407 matched that given for L plantarum. 16S rDNA sequencing was carried out to confirm the identification, which gave 99.5% homology between LP0407 and L plantarum as recorded in the Genbank database. Details of the method used are given below. The sequence is shown in SEQ ID NO: 1 in Figure 14.

### Nucleotide primers

Primers used for PCR and sequencing were synthesised using a 391 DNA synthesiser (PCR Mate Applied Biosystems, UK), using the phosphoaramidite method and purified with oligonucleotide purification cartridges (Poly-Pak™ from Glen Research, Stirling UK).

Two sequencing primers were used:- pA    Position base 9-28      AGAGTTTGATCCTGGCTCAG  Forward primer pE'   Position base 927-908   CCGTCAATTCCTTTGAGTTT   Reverse primer The sequencing primer was:-

γ    Position base 358-339   ACTGCTGCCTCCCGTAGGAG

The dried primers were re-dissolved in 200µl of sterile distilled water, diluted 1:200 in sterile distilled water and their concentration estimated spectrophotometrically at 260nm in a Pye-Unicam SP1700 (Unicam Ltd, Cambridge, UK) using a 1cm path length quartz cuvette (Sigma). The concentration was calculated according to the equation (ng/µl) = OF₂₆₀ x 200(dilution factor)/0.29, where 0.29 is a molar extinction coefficient, assumed constant.

PCR and sequencing primers were also synthesised as Ready Pure™ by Applied Biosystems, Warrington, UK. Primers were diluted to stock concentrations of 200ng/ml (for PCR reactions) or 20ng/ml (for sequencing reactions) and stored at -20°C.

### Isolation of genomic DNA

The method used is a modified version of that described by Lawson et al, (1989) for the isolation of total genomic DNA from Gram-positive organisms. Agar plates were inoculated with 100µl of an overnight culture of Lactobacillus plantarum LP0407 and incubated overnight at 37°C under appropriate conditions. Cells were harvested from the plates and re-suspended in 500µl of TES buffer (0.05M, pH 8.0). Cells were lysed by adding 10µl lysozyme (10mg/ml) and incubating the cells at 37°C for 30 minutes. This was followed by 5µl of Rnase (10mg/ml) and 5µl of proteinase K (10mg/ml) treatment for 1 hour at 65°C, and subsequently 50µl of a 20% (w/v) SDS solution was added to the cells and incubation continued at 65°C for 10 minutes. The tube contents were allowed to cool to ambient temperature before phenol extraction was performed. An equal volume (570µl) of a 1:1 phenol/chloroform mixture was added to the suspension, and gently mixed until an emulsion formed. This emulsion was centrifuged for 10 minutes at 1900 x g (Centurion 8000 Series centrifuge). The top layer (of 3) was removed into a clean eppendorf and 2.5 volumes of 100% (v/v) ethanol at -20°C were added. The solution was mixed to allow the DNA strands to precipitate. DNA was pelleted by centrifugation at 15000 x g for 5 minutes, excess ethanol was discarded, and all traces of ethanol were removed by drying in a vacuumed gyrovap (Howe). The DNA pellet was re-suspended in 60µl sterile water, and stored at -20°C.

### PCR amplification

PCR was performed using a thermal cycler (Biometra, Maidstone, UK) in a total volume of 100µl. Reactions were performed in 10 x PCR buffer (Perkin Elmer, Warrington, UK) containing 10mM deoxynucleotide triphosphates, 4ng/µl of each oligonucleotide primer, and 1ng/µl template DNA. After an initial denaturation step of 94°C for 5 minutes, tubes were placed on ice whilst 1 unit of Ampli™Tag polymerase (Perkin Elmer, Warrington, UK) was added to a final concentration of 0.02U/µl. PCR mixtures were overlaid with 2-3 drops of mineral oil (Sigma). Reactions were performed under the following conditions: 94°C for 3 minutes, followed by 30 cycles of 95°C for 1 minute, 55°C for 1 minute, 72°C for 1.5 minutes, and ending with a final extension step of 72°C for 10 minutes, before being cooled to 25°C. PCR products were analysed by agarose gel electrophoresis in 0.7% (w/v) agarose gels containing 0.5 µg/ml ethidium bromide (final concentration) and run for 25 minutes at 100V. Gels were visualised on a transilluminator (New Brunswick Scientific, Cambridge, UK).

### Purification of PCR products

PCR products were purified from primers, nucleotides, polymerases and salts using a QIAquick PCR purification kit (QIAGEN Ltd, Crawley, UK). A QIAquick spin column was placed into a provided 2ml collection tube. To bind DNA, 500µl of Buffer PB was added to the tube along with 100µl of PCR product, and centrifuged for 60 seconds at 15000 x g. The flow-through was discarded, and the spin column returned to the collection tube. The DNA was washed with 0.75ml Buffer PE and centrifuged for 60 seconds. Flow-through was again discarded and centrifuged for a further 60 seconds. The spin column was placed into a clean 1.5ml eppendorf. The DNA was eluted by adding 50µl Buffer EB to the centre of the spin column and centrifuging for 60 seconds. The average elute volume was 48µl.

### PCR direct automatic sequencing analysis

Automatic DNA sequencing was performed using an ABI DNA sequencer, model 377 (Applied Biosystems Inc, Warrington UK), and was based on the dideoxynucletotide chain termination sequencing method of Sanger et al (1977). Automatic sequencing replaces the isotope α-S³⁵ dATP used in manual sequencing for visualising sequencing products, with four different colour dye-labelled dideoxy terminators, ddATP (green), ddCTP (blue), ddGTP (black) and ddTTP (red). In addition, PCR methodology is employed in this sequencing reaction; i.e. primer/template annealing, labelling/extension and termination reactions are all incorporated into a single tube PCR reaction.

The automatic sequencing methodology used is outlined below:

### 10 Cycle Sequencing PCR reaction

This was performed using ABI prism dye terminator cycle sequencing ready reaction kit, with AmpliTaq DNA polymerase FS (Perkin Elmer, Warrington, UK). A cycle sequencing PCR reaction contained the following mixture in a 0.2ml Strip-Ease™ tube (Robbins Scientific, California, USA).

| | |
|---|---|
| Terminator ready sequence reaction mix (supplied with kit) | 4µl |
| Template PCR product (600ng) | 3µl |
| Sequencing primer (40ng/µl) | 3µl |
| Total volume | 10µl |

The amplification was carried out in the Gene Amp PCR system 9600 thermal cycler (Perkin Elmer) for 35 cycles using the following PCR program:

| | |
|---|---|
| 96°C | 15 seconds |
| 48°C | 1 second |
| 60°C | 4 minutes |

### Sequencing Product Purification

The 10µl sequencing product from the GeneAmp tube was transferred to a 0.05 ml eppendorf tube containing 1µl of 3M sodium acetate (pH 4.6) and 25 µl of 100% (v/v) ethanol. This was vortexed for 10 seconds and centrifuged at 15000 x g for 20 minutes (Centurion 8000 Series centrifuge). The supernatant was then drained, and 200µl of 70% (v/v) ethanol was added to the pellet and centrifuged for 15 minutes at 15000 x g. The supernatant was drained and the sequencing product dried in a gyrovap (VA Howe, Banbury, UK) for 15 minutes. 1µl of 0.05M EDTA and 5µl of formamide was added to each sample and vortexed for 10 seconds. Each sample was heated on a PCR block (Perkin Elmer) for 5 minutes at 90°C and immediately placed on ice before loading.

### Sequencing gel preparation and electrophoresis

Acrylamide gel preparation and electrophoresis was carried out according to manufacturers' instructions. 100ml gels were prepared using the Sequagel system (National Diagnostics, Atlanta, USA) and an ABI automatic sequencing gel casting unit. Each 100ml of sequencing gel contained 24ml Sequagel concentrate, 66ml Sequagel diluent and 10ml Sequagel buffer. 35µl of TEMED and 150µl of 20% APS were added and mixed carefully in a beaker to prevent the introduction of air bubbles. The gel was poured into the gel casing mould and left to set for 1 hour at room temperature. The gel was mounted onto an ABI 377 DNA sequencer and pre-run in 1 x TBE buffer for 1 hour before loading 6µl of each of the 24 sequencing samples. After loading all the samples, the gel was run for 14 hours at a constant power of 50W, and the sequencing data scanned into a computer using a laser scanner, which reads the four different fluorescent terminators.

### Sequencing data analysis

Sequences were entered manually and stored in individual files on a VAX/VMS computer (VAX 4000-100A). Sequences were edited and checked manually to check for computer algorithm errors in spacing.

### References

Lawson PA, Gharbia SE, Shah HN and Clark DR (1989) Recognition of *Fusobacterium nucleatum* subgroups FN-1, FN-2 and FN-3 by ribosomal RNA gene restriction patterns. *Microbiology Letters* **65** 41-45.
Sanger F, Nicklen S, and Coulson AR (1977) DNA sequencing with chain termination inhibitors. *Proceedings of the National Academy of Sciences of the USA* 74 5463-5467

### EXPERIMENT 2: INHIBITION OF FOOD POISONING BACTERIA

The inhibition of food poisoning bacteria was demonstrated by growing bacteria in the presence of L plantarum LP0407 in the presence or absence of prebiotic oligosaccharides, using co-culture and batch fermentation techniques.

The food poisoning organisms used were as follows:-
Escherichia coli NCIMB 9517, an enteropathogenic strain similar to E coli O157:H7, but which does not produce verocytotoxin. E. coli is a cause of infections in the large intestine, and E. coli O157:H7 has a very low infective dose and may cause a fatal infection.
Campylobacter jejuni NCTC 11351, a strain isolated from a human source. C jejuni also causes infections in the large intestine, and it is the major cause of food poisoning in the United Kingdom.
Salmonella enteritidis 4444, a strain isolated from a human source that is similar to a strain known to cause food poisoning. S. enteritidis causes food poisoning by infecting the small intestine.

The prebiotic oligosaccharides used and the control were as follows:-
FOS oligofructose
   (short chain fructo-oligosaccharides produced by partial hydrolysis of inulin, consisting of a mixture of GFₙ (∼70%) and Fₘ oligomers with an average degree of polymerisation of 4-5, where G is α(1-2) linked D-glucose and F is β(2-1) linked D-fructose)
XOS xylo-oligosaccharides
   (short chain xylo-oligosaccharides, consisting of Xₙ oligomers of degree of polymerisation 2-9, where X is β(1-4) linked xylopyranose)
FOS:XOS a 50:50 mixture of oligofructose and xylooligosaccharides
IN:FOS a 20:80 mixture of inulin and oligofructose
   (inulin : longer chain fructo-oligosaccharides, consisting of GFₙ oligomers with an average degree of polymerisation of 10 (range 2-60), where G is α(1-2) linked D-glucose and F is β(2-1) linked D-fructose)
   STAR starch (control - no prebiotic effect)

The oligofructose was obtained from Orafti, Belgium (Raftilose TM); the xylo-oligo-saccharides were obtained from Suntory, Japan (Xylo-oligo 35P TM); and the inulin was obtained from Orafti, Belgium (Raftiline TM).

### (i) Co-culture experiments

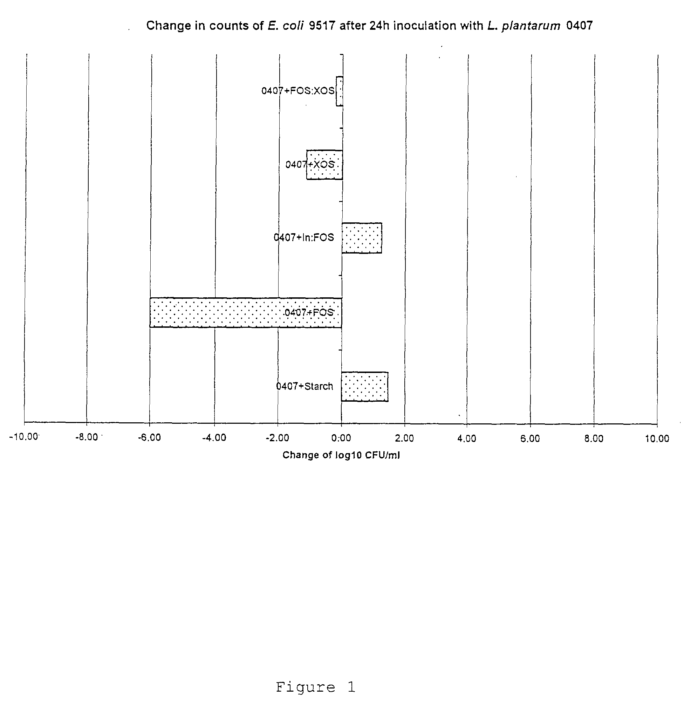

L plantarum LP0407 was grown overnight on MRS broth and the food poisoning bacteria were grown overnight on Mueller Hinton broth. 1mL of L plantarum LP0407 culture and 1mL of a food poisoning bacteria culture (∼10⁸-10⁹ cells) were inoculated into basal medium containing 1% of one of the oligosaccharides or of the starch control and incubated anaerobically. Samples were removed at 0, 3, 6, 9, and 24 hours and a dilution series prepared and plated out on the appropriate agar. The plates were incubated for 48 hours and the colonies enumerated.
The results of the trials are given in the figures as follows:-
- Figure 1: E coli - change in log₁₀ counts after 24 hours

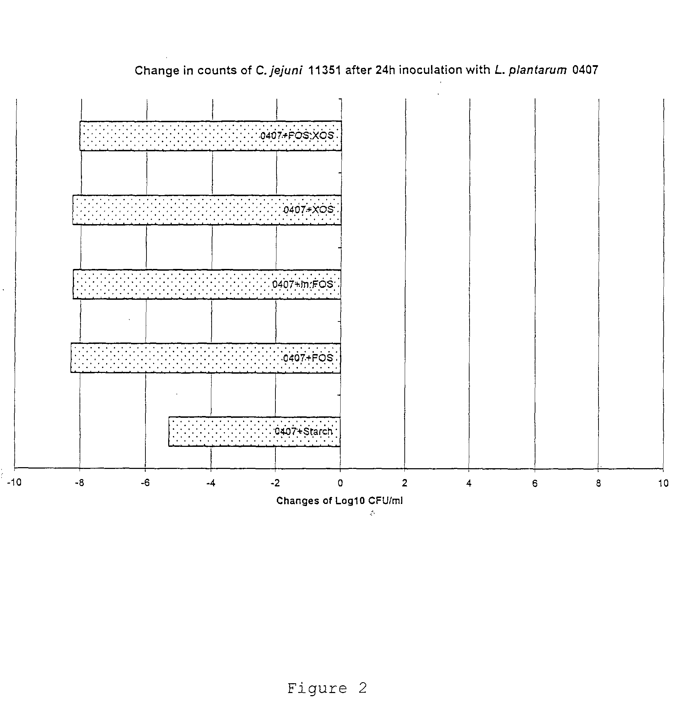

- Figure 2: C jejuni - change in log₁₀ counts after 24 hours

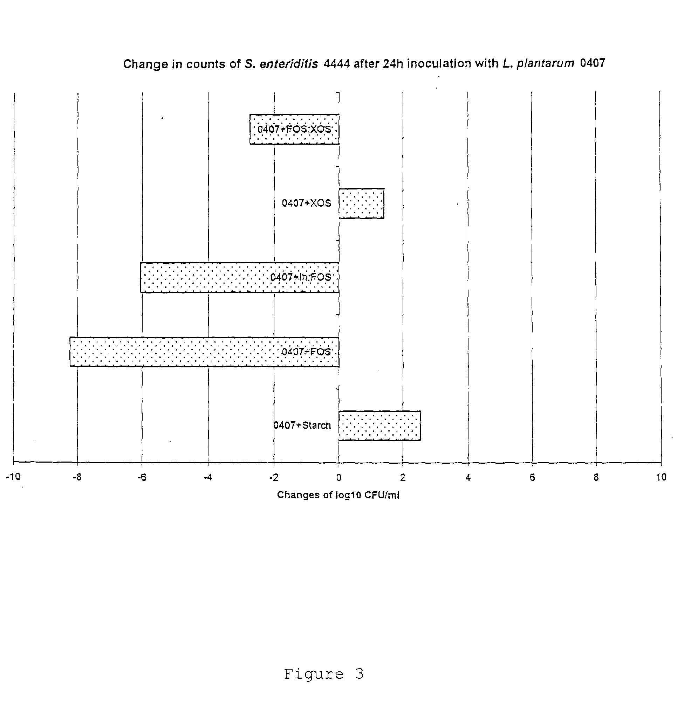

- Figure 3: S enteritidis - change in log₁₀ counts after 24 hours

The results can be summarised as follows:-
(1) E coli was only inhibited by L plantarum LP0407 when grown in the presence of oligofructose. After 24 hours there was a 6 log decrease in the count of E coli in the presence of FOS, but there was no significant change in counts in the presence of XOS, FOS:XOS, IN:FOS or STAR (control).
(2) C jejuni was inhibited by L plantarum LP0407 in the presence of FOS, XOS, FOS:XOS or IN:FOS; after 24 hours there was an 8 log decrease in counts in each case. There was also a 5 log decrease in counts of C jejuni in the presence of STAR (control).
(3) S enteritidis was only inhibited by L plantarum LP0407 in the presence of FOS or IN:FOS. After 24 hours there was an 8 log decrease in the count in the presence of FOS and a 6 log decrease in the count in the presence of IN:FOS, but there was no significant decrease in counts in the presence of XOS, FOS:XOS or STAR (control).

### (ii) Batch Fermentation Experiments

Batch culture fermentation systems containing basal growth medium were inoculated with a 10% w/v faecal homogenate, the faecal sample being obtained from a healthy volunteer who had no history of gastro-intestinal disturbance and who had not taken antibiotics for the previous three months. The fermentation systems were incubated at 37°C and were continually stirred and sparged with oxygen-free nitrogen. Fermentations were carried out either with the pH controlled at pH 7 or without pH control.

Further additions were made as follows, with the pathogen being either Escherichia coli NCMB 9517 or Campylobacter jejuni NCTC 11351:-

| **Code** | **Prebiotic** | **Probiotic (LP0407)** | **Pathogen** |
|---|---|---|---|
| A | - | - | - |
| B | FOS (1% w/v) | - | - |
| C | FOS (1% w/v) | 10⁹ cfu | - |
| D | - | - | 10⁹ cfu |
| E | FOS (1% w/v) | - | 10⁹ cfu |
| F | FOS (1% w/v) | 10⁹ cfu | 10⁹ cfu |

Samples of 1mL were taken from each fermentor after 0, 6, 10 and 24 hours incubation and serially diluted to 10⁻⁷ in an anaerobic cabinet with half-strength peptone water. Replicate plates were then inoculated with 20µL diluent samples, and incubated under aerobic, anaerobic or microaerophilic conditions as appropriate. The media used were Rogosa agar for lactobacilli, MacConkey No.3 agar for coliforms, and Campylobacter blood-free specific supplemented agar for C jejuni. After incubation differential counts were made based on colony morphotypes and microscopy.

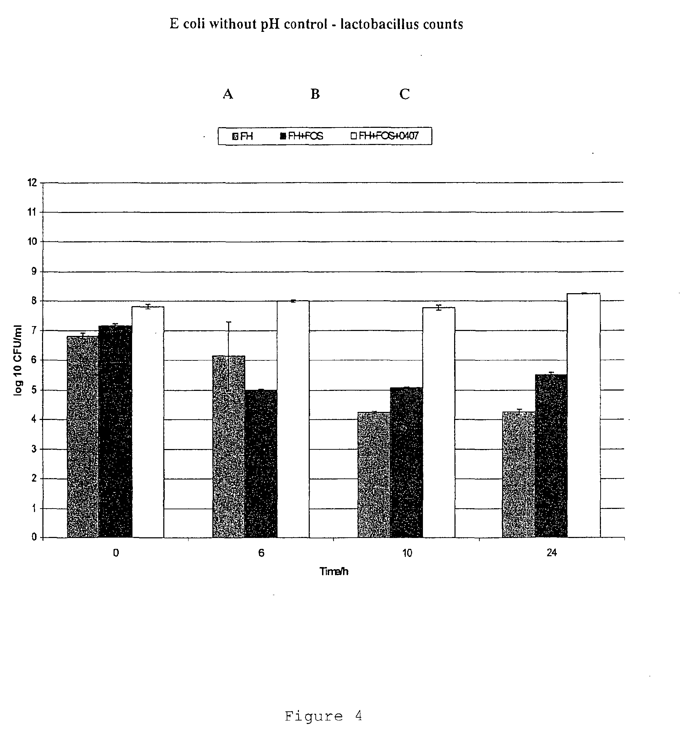

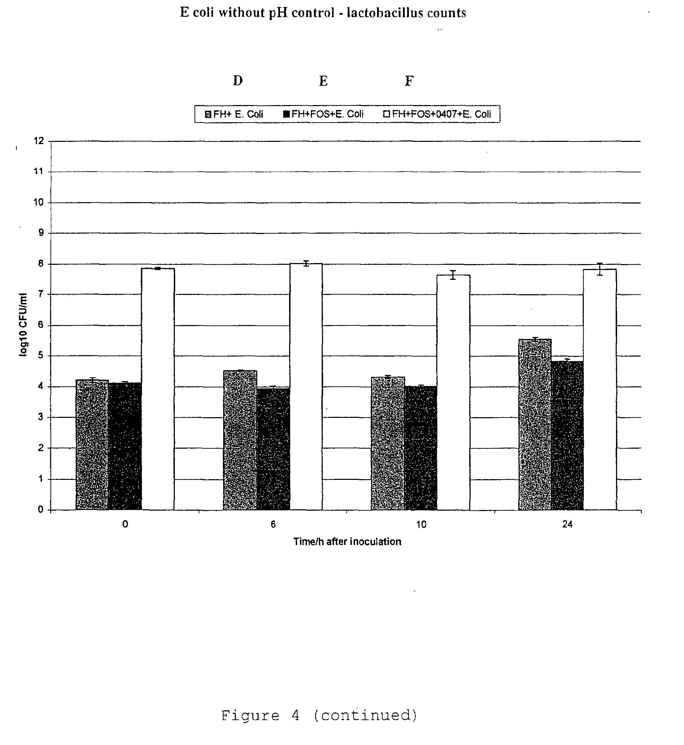

The results are shown in the figures as follows:-
- Figure 4: E coli without pH control - lactobacilli counts

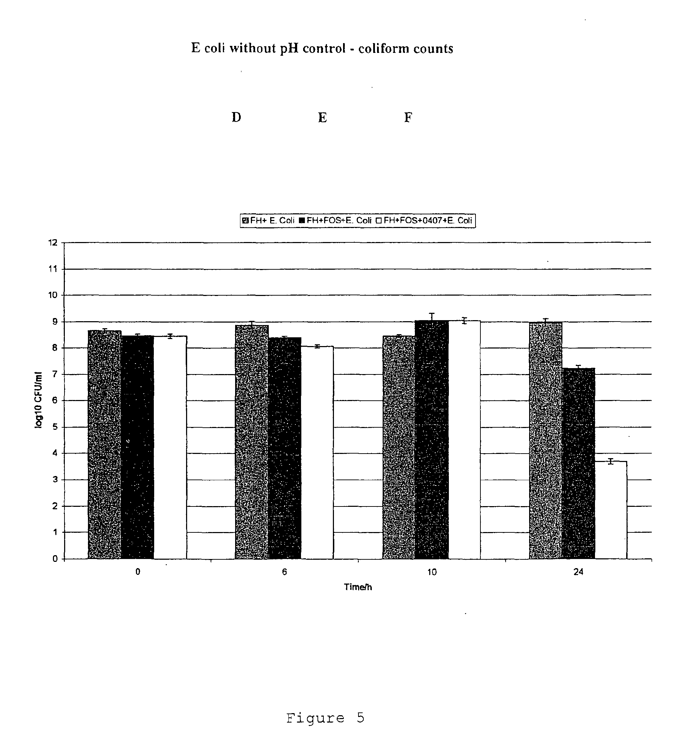

- Figure 5: E coli without pH control - coliform counts

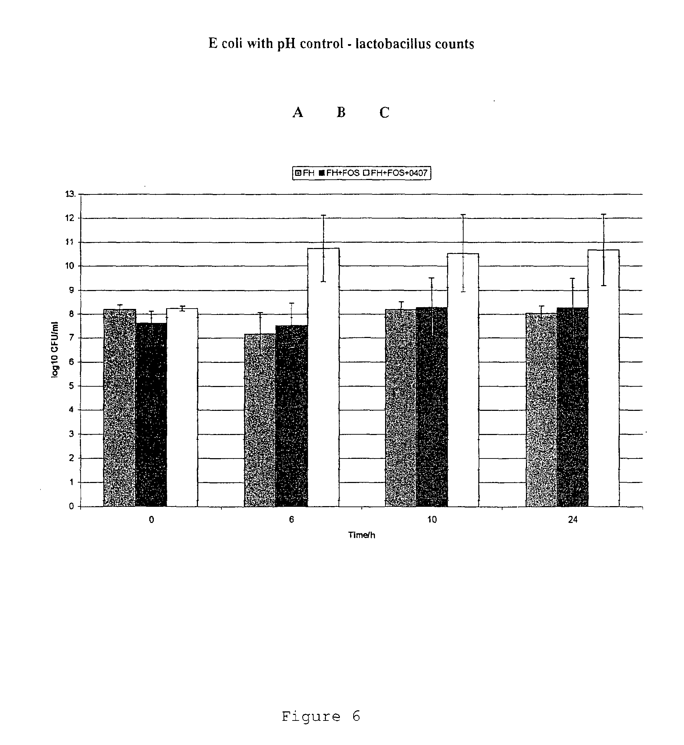

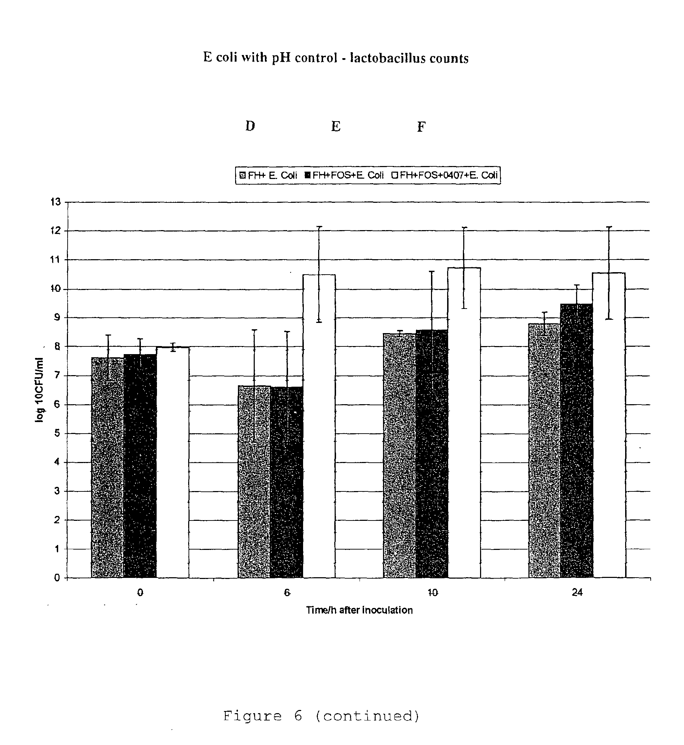

- Figure 6: E coli with pH control - lactobacilli counts

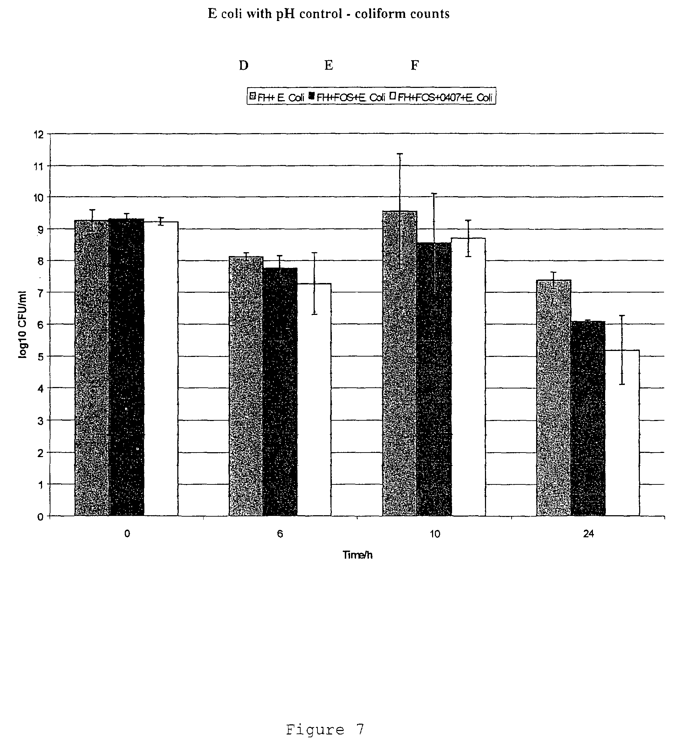

- Figure 7: E coli with pH control - coliform counts

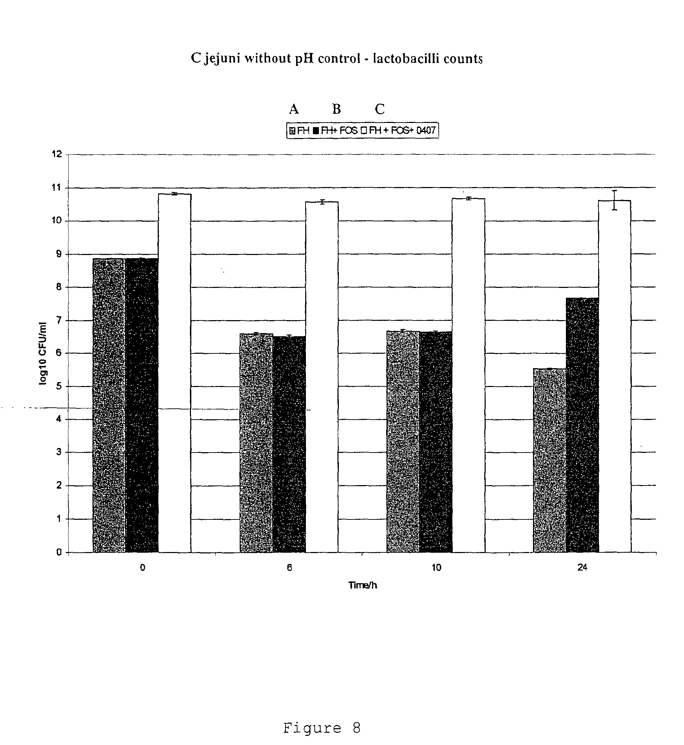

- Figure 8: C jejuni without pH control - lactobacilli counts

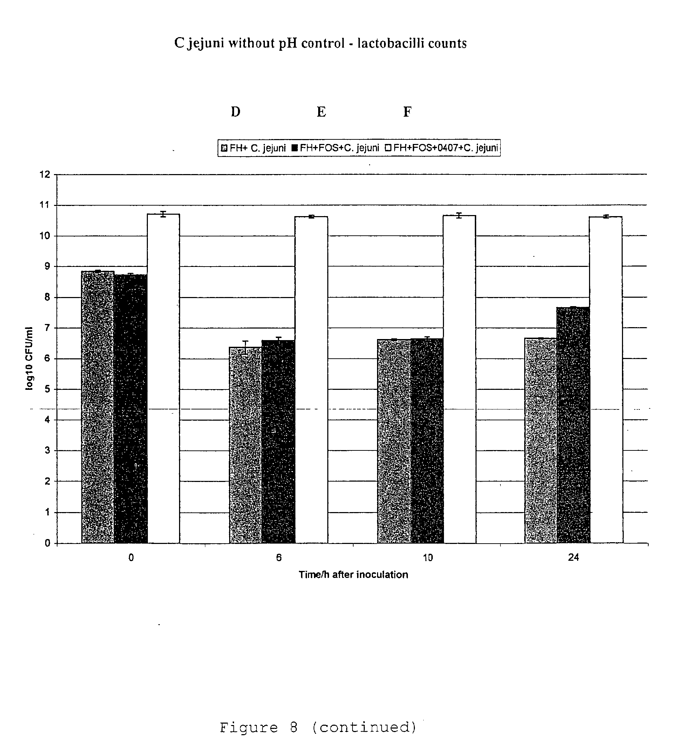

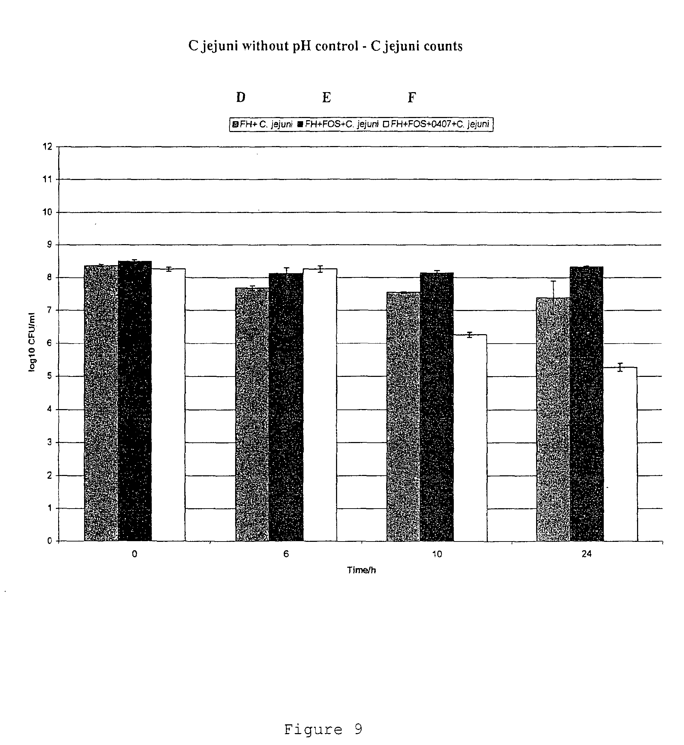

- Figure 9: C jejuni without pH control - C jejuni counts

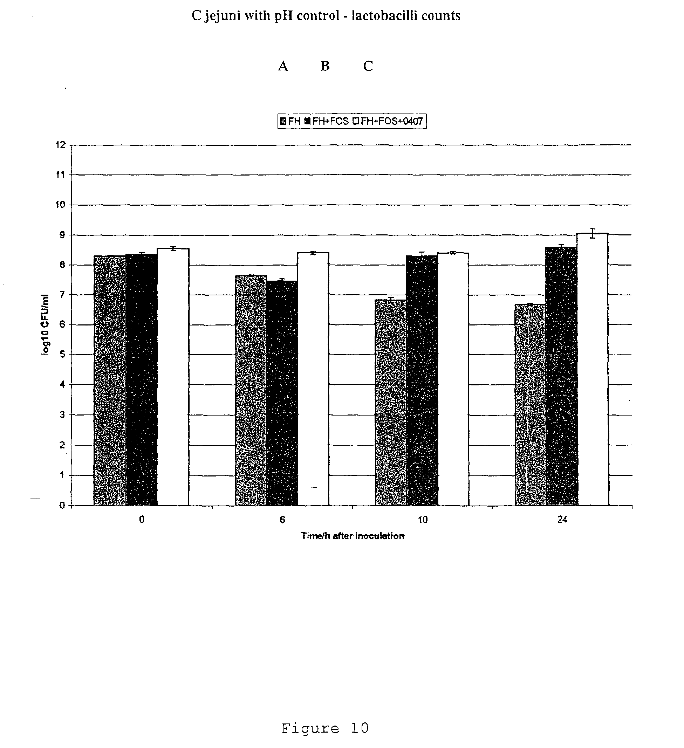

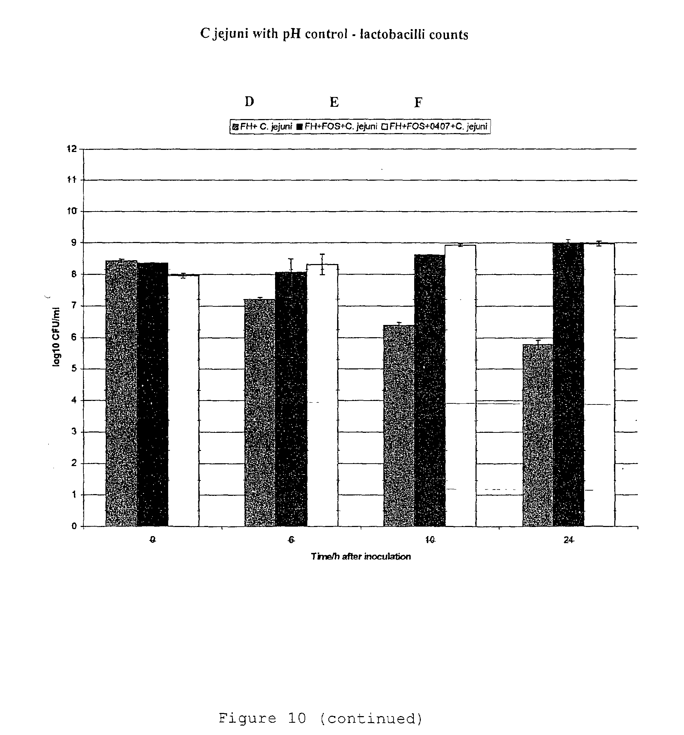

- Figure 10: C jejuni with pH control - lactobacilli counts

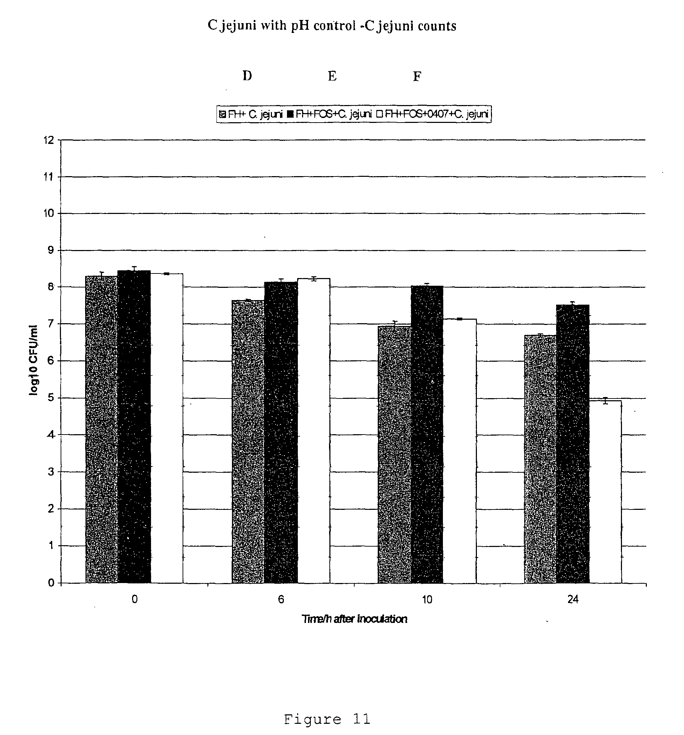

- Figure 11: C jejuni with pH control - C jejuni counts

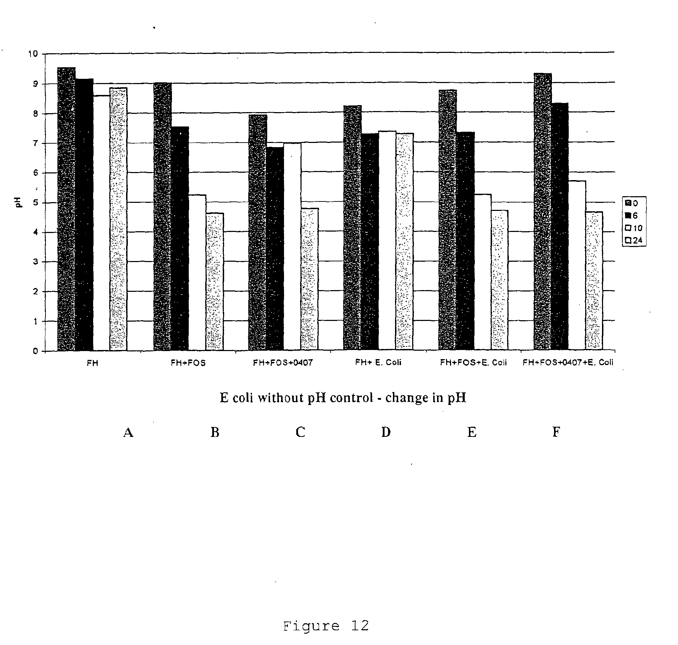

- Figure 12: E coli without pH control - change in pH

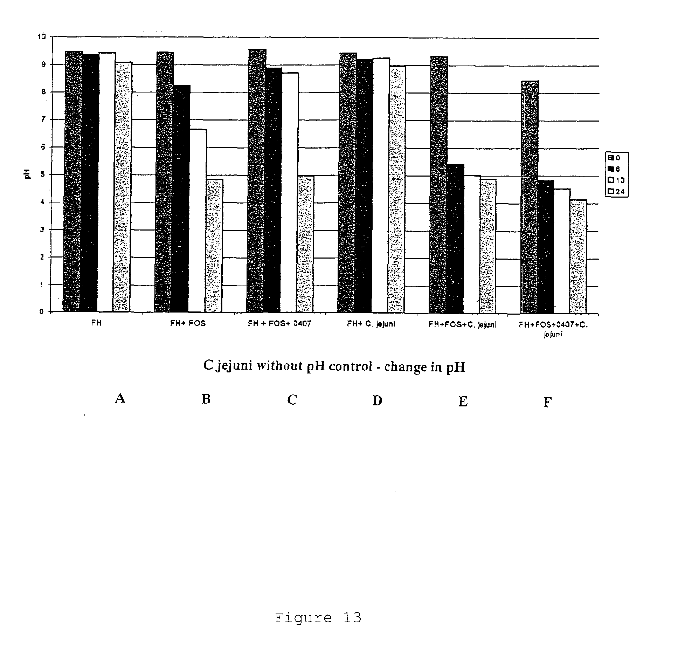

- Figure 13: C jejuni without pH control - change in pH

The results can be summarised as follows:-
(1) Lactobacilli counts were initially high in the fermentors to which L plantarum LP0407 was added, and after 24 hours were maintained with no pH control or increased (>2 log increase) with pH control. In the fermentors without added L plantarum LP0407, initial lactobacilli counts were generally lower and after 24 hours were either maintained or decreased (or increased if initially very low), with or without pH control.
(2) Coliform counts showed little change without addition of prebiotic or probiotic, fell after 24 hours in the presence of prebiotic (2-3 log decrease), and fell more markedly after 24 hours in the presence of both prebiotic and probiotic (4-5 log decrease). The presence or absence of pH control made little difference to these results.
(3) C jejuni counts fell in the fermentors with no additions and with added prebiotic, but fell markedly (3 log decrease) in the fermentor with added prebiotic and probiotic. The presence or absence of pH control had little effect on C jejuni counts.
(4) The pH in the fermentors without pH control had begun to fall by 6 hours of incubation, except in the fermentors with no addition of prebiotic or probiotic, where it was fairly stable (<1 pH unit decrease) up to 24 hours. After 24 hours the pH in the fermentors with added prebiotic or prebiotic plus probiotic showed decreases of 3.5-5 pH units. Pathogen counts did not begin to fall until after 10 hours, and in most cases showed no marked fall until 24 hours.

The following Examples illustrate food products according to the invention.

### EXAMPLE 1 - FROMAGE FRAIS BASE

| | |
|---|---|
| Skimmed milk | 85.0% |
| Functional milk protein | 9.0% |
| Inulin / Oligofructose | 3.0% (20/80) |
| 40% fat cream | 3.0% |

The dry ingredients and the cream were mixed into the skim milk at 10°C and allowed to hydrate for 1 hour. The mixture was then heated to 60°C, homogenized at 3000psi, pasteurized at 95°C for 5 minutes and cooled to 41°C. Streptococcus thermophilus was added as the starter culture, together with L plantarum 0407 (at a level of 10¹³ cells per 1000 litres of product), and fermentation was allowed to proceed until the pH reached 4.5 when the curd was pitched. The curd was then cooled to 20°C in a plate heat exchanger, when it was ready for use in making a fromage frais dessert.

### EXAMPLE 2 - PROTEIN CONCENTRATE BASE

| | |
|---|---|
| Skimmed milk | 67.0% |
| Water | 21.0% |
| Functional Milk Protein | 8.0% |
| Inulin / oligofructose | 4.0% (20/80) |

The dry ingredients were mixed into the skim milk and water and allowed to hydrate by applying slow speed agitation for 1 hour. The mixture was pasteurised at 90°C for 6 minutes, homogenised at 500psi and cooled to 36°C. After transfer to a fermentation tank, a starter culture consisting of L lactis, S thermophilus and L plantarum 0407 (at a level of 10¹³ cells per 1000 litres of product) was inoculated into the mixture. Fermentation was allowed to proceed until the pH reached 4.7, when the mixture was agitated for 10 minutes and cooled in a plate heat exchanger to 8°C. It was then ready for use in the manufacture of food products.

### EXAMPLE 3 - CULTURED CREAM DRESSING FOR COTTAGE CHEESE

| | |
|---|---|
| Skimmed milk | 64.0% |
| 40% fat cream | 25.0% |
| Xylo-oligosaccharides | 6.0% |
| Skimmed milk powder | 4.4% |
| Salt | 0.6% |

The cream was pasteurised at 80°C for 15 seconds and the skimmed milk was pasteurised at 72°C for 15 seconds. They were then mixed together and the skimmed milk powder and xylooligosaccharides were added, followed by gentle agitation for 10 minutes. The mixture was then pasteurised at 86°C for 60 seconds and subjected to a two stage homogenisation at 2000psi (first stage) and 500psi (second stage), followed by cooling to the fermentation temperature of 40°C. A starter culture of acidifying mesophiles plus L plantarum 0407 (at a level of 10¹² cells per 1000 litres of product) was then added and fermentation was allowed to proceed. At pH 4.8 the salt was added and the dressing was agitated for 15 minutes. It was then cooled to 8°C and stored, ready to be used to dress cottage cheese curd.

### EXAMPLE 4A - BIO YOGHURT BASE (VERY LOW FAT)

| | |
|---|---|
| Skimmed milk | 91.0% |
| Oligofructose | 6.0% |
| Functional milk protein | 3.5% |
| Hydrocolloid stabiliser | 0.5% |

### EXAMPLE 4B - BIO YOGHURT BASE (LOW FAT)

| | |
|---|---|
| Skimmed milk | 91.0% |
| Oligofructose | 4.0% |
| Functional milk protein | 3.5% |
| 50% fat cream | 2.0% |
| Hydrocolloid stabiliser | 0.5% |

The dry ingredients and the cream at 5°C (if present) were mixed into skimmed milk at 5°C. After hydration for a minimum of 1 hour at 10°C, the mixture was preheated to 60°C and homogenised at 3000psi. Following pasteurisation at 95°C for 5 minutes and cooling to the incubation temperature of 41°C, a standard bio yoghurt culture (S thermophilus, L bulgaricus, L acidophilus, B bifidum) was added, along with L plantarum 0407 (at a level of 10¹³ cells per 1000 litres of product). When the pH reached 4.55, the product was cooled to 14°C in a plate heat exchanger and stored ready for packing and addition of fruit.

## Claims

1. A food product containing a combination of (i) a probiotic Lactobacillus plantarum bacterium and (ii) at least one prebiotic oligosaccharide, in an amount so that the combination is capable of inhibiting the growth of food poisoning bacteria.

2. A food product according to claim 1, wherein the Lactobacillus plantarum bacterium has a 16S rDNA sequence comprising (i) a sequence as shown in SEQ ID NO: 1 or (ii) a sequence that is a mutation or variant thereof and which does not substantially reduce the ability of the bacterium to inhibit the growth of food poisoning in the presence of at least one prebiotic oligosaccharide.

3. A food product according to claim 1 or claim 2, wherein the Lactobacillus plantarum bacterium is Lactobacillus plantarum strain LP0407 (NCIMB No. 41034).

4. A food product according to any one of claims 1 to 3, wherein the at least one prebiotic oligosaccharide comprises a mixture of prebiotic oligosaccharides.

5. A food product according to any one of the preceding claims, wherein the at least one prebiotic oligosaccharide is selected from the group consisting of fructo-oligosaccharides, xylo-oligosaccharides and mixtures thereof.

6. A food product according to claim 5, wherein the at least one prebiotic oligosaccharide comprises 50% by weight or more of oligofructose.

7. A food product according to any one of the preceding claims, wherein the at least one prebiotic oligosaccharide is present at a amount of up to 10% by weight based on the total weight of the food product.

8. A food product according to any one of the preceding claims, wherein the probiotic Lactobacillus plantarum bacterium is present in the food product at an amount of at least 10¹² cells per 1000 litres or per 1000 kilograms.

9. A food product according to any one of the preceding claims, wherein the combination of the probiotic Lactobacillus plantarum bacterium and the at least one prebiotic oligosaccharide is homogeneously dispersed within the food product.

10. A food product according to any one of the preceding claims, which product comprises a cultured or non-cultured dairy product.

11. A food product according to claim 10, wherein the cultured dairy product is a yoghurt or cheese product.

12. A combined preparation for inhibiting the growth of food poisoning bacteria comprising:
(i) a probiotic Lactobacillus plantarum bacterium; and
(ii) at least one prebiotic oligosaccharide.

13. A combined preparation according to claim 12 for use in a food product.

14. A combined preparation according to claim 12 or claim 13, wherein the at least one prebiotic oligosaccharide comprises a mixture of prebiotic oligosaccharides.

15. A combined preparation according to any one of claims 12 to 14, wherein the prebiotic Lactobacillus plantarum bacterium or the at least one prebiotic oligosaccharide or mixture of oligosaccharides are as defined in claims 2 to 5.

16. An isolated Lactobacillus plantarum bacterium for use in the preparation of a food product as defined in any one of claims 1 to 11 or a combined preparation as defined in any one of claims 12 to 15.

17. An isolated Lactobacillus plantarum bacterium according to claim 16, which has a 16S rDNA sequence comprising (i) a sequence as shown in SEQ ID NO: 1 or (ii) a sequence that is a mutant or variant thereof and which does not substantially reduce the ability of the bacterium to inhibit the growth of food poisoning bacteria.

18. An isolated Lactobacillus plantarum bacterium according to claim 17, which is Lactobacillus plantarum strain LP0407 (NCIMB No. 41034).

19. Use of a combined preparation as defined in any one of claims 12 to 15, for the manufacture of a composition for inhibiting the growth of food poisoning bacteria.

20. Use of a combined preparation as defined in any one of claims 12 to 15, for the preparation of a food product for inhibiting the growth of food poisoning bacteria.

21. Use according to claim 19 or claim 20, wherein the food poisoning bacteria are those that cause food poisoning by growing in the large intestine.

22. Use according to claim 21, wherein the inhibition of the growth of food poisoning bacteria is in the human large intestine.

23. Use of an oligosaccharide for the preparation of a food product as defined in any one of claims 1 to 11 or a combined preparation as defined in any one of claims 12 to 15.

24. Use of an isolated Lactobacillus plantarum bacterium as defined in any one of claims 16 to 18 in a food product as defined in any one of claims 1 to 11 or a combined preparation as defined in any one of claims 12 to 15.

25. A method for making a combined preparation as defined in any one of claims 12 to 15, comprising a step of introducing the probiotic Lactobacillus plantarum bacteria with a mixture containing the at least one prebiotic oligosaccharide.

26. A method for making a food product as defined in any one of claims 1 to 11, comprising the step of contacting the probiotic Lactobacillus plantarum bacteria with a mixture containing the at least one prebiotic oligosaccharide.
